# EUROPÄISCHE PATENTSCHRIFT

(11) **EP 0 514 320 B1**
(45) Veröffentlichungstag und Bekanntmachung des Hinweises auf die Patenterteilung: **15.11.1995**
(21) Anmeldenummer: 92810263.1
(22) Anmeldetag: 07.04.1992
(51) Int. Cl.: D03D 51/34, G01N 33/36, B65H 63/032

(54) **Elektrostatischer Schussfadenwächter und Webmaschinen mit einem derartigen Schussfadenwächter**
Electrostatic weft-detector and loom with such weft-detector
Détecteur de trame électrostatique et métier à tisser pourvu d'un tel détecteur de trame

(30) Priorität: 15.05.1991 CH 1449/91
(43) Veröffentlichungstag der Anmeldung: 19.11.1992
(73) Patentinhaber: SULZER RÜTI AG, CH-8630 Rüti (CH)
(72) Erfinder: De Jager, Godert, Dr., CH-8121 Englen (CH)
(74) Vertreter: Heubeck, Bernhard

(56) Entgegenhaltungen:
- EP-A- 0 036 314
- DE-A- 2 140 812
- DE-A- 2 435 375
- DE-A- 2 758 403
- FR-A- 2 125 156
- FR-A- 2 376 058

## Beschreibung

Die Erfindung betrifft einen Schussfadenwächter für Webmaschinen, dessen Sensorelement berührungslos auf elektrische Ladung des Schussfadens anspricht bzw. empfindlich ist sowie Webmaschinen bei denen der erfindungsgemässe Schussfadenwächter Verwendung findet.

Ein Konzept für Schussfadenwächter ist z.B. aus der DE-PS 27 58 403 bekannt. Verschiedene Ausführungsformen elektrostatischer Wandler sind darin offenbart. Diese Sensoren werden dort hauptsächlich bei Luftwebmaschinen eingesetzt. Der Schussfaden wird während des Abtragens vom Schussfadenvorrat aufgrund der auftretenden Reibung als auch während des Schusseintrags aufgrund der Reibung mit der Luft elektrisch geladen. Der elektrostatische Schussfadenwächter registriert die Anwesenheit einer sich vorbeibewegenden und auf diese Weise elektrisch geladenen Texilfaser, insbesondere kann auch der Durchgang der Spitze eines eingetragenen Schussfadens detektiert werden. Schussfadenwächter werden im Schusskanal der Webmaschine eingesetzt. Die bekannten Ausführungsformen sind relativ gross und schwer und sind häufig in Form einer Konfusorlamelle ausgeführt. Die heute üblichen hochtourigen Luftwebmaschinen mit einer entsprechend hohen Anschlagszahl des Webblattes bewirken hohe Vibrations- und Beschleunigungsbelastungen auf die bekannten Schussfadenwächter, so dass die bekannten Ausführungsformen für den Einsatz an Luftwebmaschinen nicht mehr geeignet sind oder die resultierenden elektrischen Signale stark verrauscht sind.

Die Erfindung hat die Aufgabe, einen berührungslosen Schussfadenwächter, basierend auf elektrostatischem Funktionsprinzip herzustellen, der sich insbesondere für den Einsatz in hochtourigen Luftwebmaschinen eignet.

Diese Aufgabe wird mit einem Schussfadenwächter mit den Merkmalen des unabhängigen Anspruchs 1 gelöst. Die abhängigen Ansprüche beziehen sich auf besonders vorteilhafte Ausführungsformen der Erfindung.

Der Schussfadenwächter detektiert mittels eines plattenartig geschichteten Sensorelementes berührungslos eine, durch einen sich vorbeibewegenden Schussfaden bewirkte, Potentialverschiebung im Fühler. Das Sensorelement ist, mit dem Fühler dem Schussfadeneintrag zugewandt, zwischen die Lamellen des Webblattes einführbar. Der Schussfadenwächter und das Sensorelement ist längs des Webblattes, abhängig vom Raster der Lamellen, variabel positionierbar und wird üblicherweise je nach Webbreite unmittelbar ausserhalb der Kettfäden plaziert.

Das Sensorelement ist aus plattenartigen Schichten geringer Masse aufgebaut. Die äussersten, aufliegenden zwei Schichten sind elektrisch leitend und dienen zur Abschirmung elektrischer Felder. Dazwischen liegt jeweils eine plattenartige Schicht aus Isolationsmaterial und in der Mitte befindet sich der eigentliche Fühler, ein vorzugsweise als offene Schlinge gebildeter, flächiger elektrischer Leiter. Das Sensorelement, insbesondere die Schichten des Fühlers, stehen vorzugsweise senkrecht zur Schusseintragsrichtung. Die dem Schusskanal zugewendete Kante des Sensorelementes folgt vorzugsweise den Konturen des Schusskanals im Webblatt, wie z.B. derjenigen eines Profilblattes. Das Sensorelement lässt sich zwischen die Lamellen des Webblattes einfügen, wodurch sich eine Abstützung für das Sensorelement für die in Schusskanalrichtung wirkenden Kräfte, Vibrationen und Beschleunigungen ergibt. Der steife, massearme schichtförmige Aufbau ist sehr formstabil und verhindert gegenseitige Relativbewegungen der plattenartigen leitenden Schichten und reduziert somit das bei hohen Vibrations- und Beschleunigungsbelastungen auftretende Rauschen des elektrischen Signales, welches durch Kapazitätsänderungen verursacht wird.

Der ganze Schussfadenwächter ist vorzugsweise als mehrschichtige Platine ausgestaltet, wobei die mittlere leitende Schicht nebst dem Fühler auch weitere Leiterbahnen für elektronische Komponenten, inbesondere einem Ladungsverstärker, aufweisen kann. Das Sensorelement ist sehr kostengünstig produzierbar. Wird ein integrierter Schaltkreis, wie z.B. ein Ladungsverstärker, unmittelbar auf die mittlere Schicht aufgebracht, so ist ein sehr dünner Schussfadenwächter realisierbar, der durch die beiden äusseren, überdeckenden plattenartigen leitenden Schichten elektrisch abgeschirmt ist. Werden als elektronische Bauteile grössere Komponenten verwendet, so können die Leiterbahnen auch aus einem Teilbereich der äussersten, elektrisch leitenden Schicht des Schussfadenwächters gebildet sein und die elektronischen Bauteile darauf aufgebracht werden. Zusätzlich ist dann ein abschirmendes metallisches Gehäuse notwendig, das die elektronischen Bauteile sowie die Leiterbahnen gegen aussen abschirmt.

Der Schussfadenwächter lässt sich mit einer Befestigungsvorrichtung am Webblatt oder an der Weblade fixieren. Er wird vorzugsweise zwischen die Lamellen des Webblattes eingefügt und ist längs des Webblattes, abhängig vom Raster der Lamellen, variabel positionierbar, derart, dass das Sensorelement ausserhalb der Webbreite zu liegen kommt. Das Webblatt kann somit für unterschiedliche Webbreiten auf Originallänge belassen werden und der Sensor so positioniert werden, dass er z.B. ausserhalb der momentanen Webbreite unmittelbar an die Kettfäden angrenzt. Je nach Breite des Webgutes kann der Sensor daher längs des Webblattes in eine optimale Position gebracht werden.

Die Erfindung hat den Vorteil, dass der Schussfadenwächter ohne Änderung des funktionellen Aufbaus der Webmaschine längs des Webblattes weitgehend frei wählbar angeordnet werden kann.

Ein weiterer Vorteil des erfindungsgemässen Sensorelementes ist, dass es gegen Verschmutzung unempfindlich ist.

Nachfolgend werden anhand der Figuren Ausführungsbeispiele der Erfindung näher erläutert. Es zeigen:
- Fig. 1: eine in Richtung des Schusskanals auseinandergezogene perspektivische Ansicht eines Schussfadenwächters;
- Fig.2: ein schematischer Ausschnitt eines Webblattes in einer Luftwebmaschine zur Illustration der Lage des Schussfadenwächters im Profilblatt;
- Fig.3: ein perspektivischer Ausschnitt eines Sensorelementes gemäss Fig. 1;
- Fig.4: ein schematischer Querschnitt durch das Sensorelement in Fig. 3 zur Illustration des schichtförmigen Aufbaus;
- Fig. 5: eine schematische Ansicht der mittleren leitenden Schicht des Sensorelementes;
- Fig. 6: schematisch die perspektivische Ansicht eines Schussfadenwächters mit Befestigungsvorrichtung am Webblatt;
- Fig. 7: schematisch die perspektivische Ansicht eines Schussfadenwächters mit Befestigung an der Weblade; und
- Fig. 8: eine weitere perspektivische Darstellung für einen Schussfadenwächter mit Befestigungsvorrichtung.

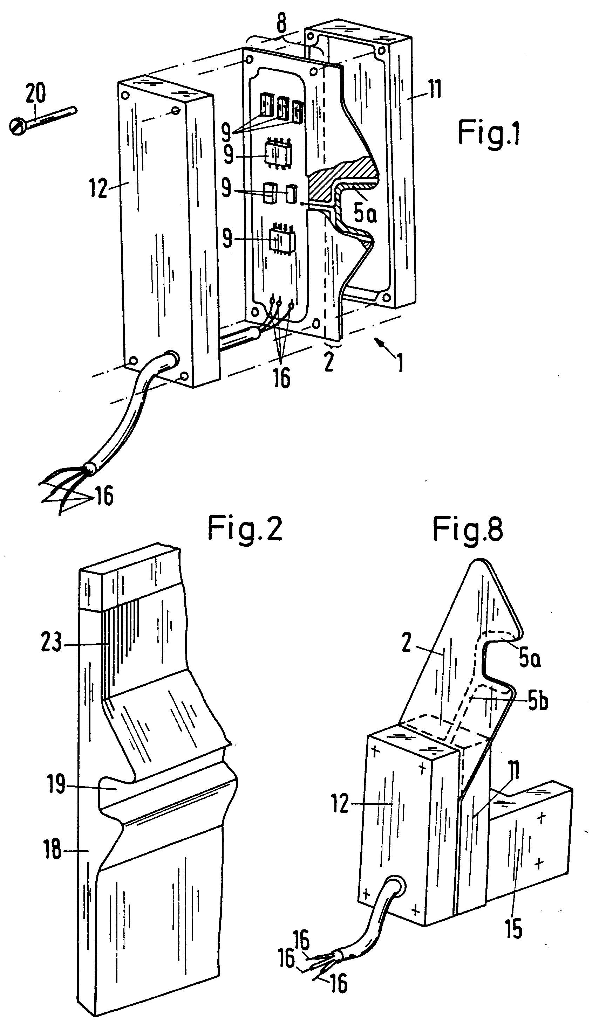

In Fig. 1 ist eine perspektivische Ansicht des Schussfadenwächters 1 mit dessen Teilkomponenten gezeigt. Der Schussfadenwächter 1 enthält ein Sensorelement 2 sowie eine Trägerplatine 8 für elektronische Komponenten 9. Elektrisch leitende Gehäusedeckel 11, 12 schützten den dadurch umschlossenen Innenraum des Schussfadenwächters 1 vor elektrischen Feldern, vor mechanischen Einflüssen und ermöglichen zusammen mit den als Schrauben ausgeführten Befestigungsmitteln 20 sowie einer Befestigungsvorrichtung 15 den Schussfadenwächter 1 an der Weblade 22 oder am Webblatt 18 zu fixieren.

Fig. 2 zeigt ein Webblatt 18, das üblicherweise aus den angedeuteten Lamellen 23 aufgebaut ist. Als Beispiel ist das Webblatt einer Luftwebmaschine mit integriertem Schusskanal 19 dargestellt.

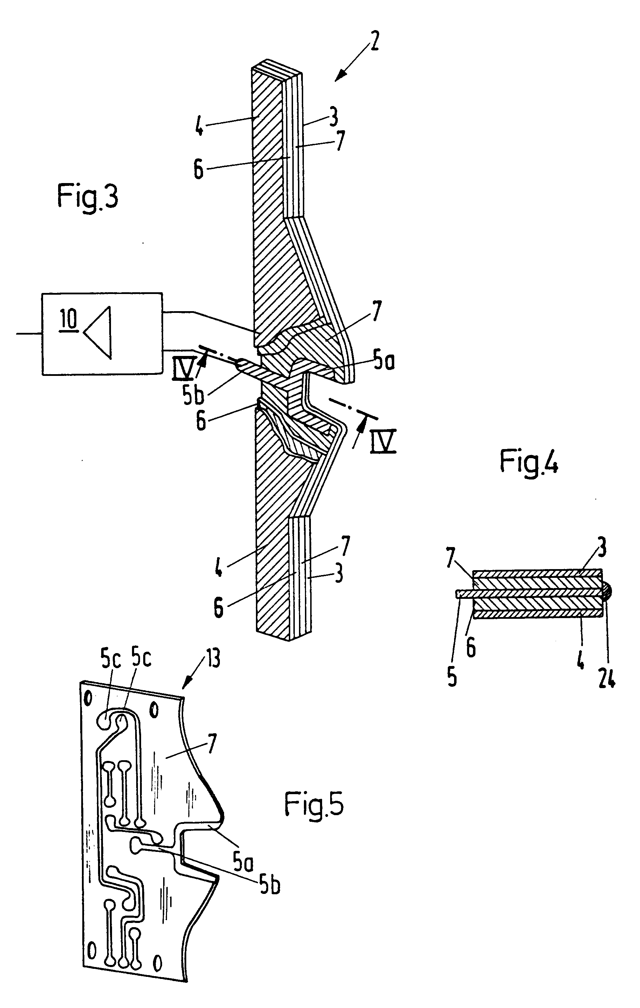

In Fig. 3 erfasst der Fühler 5a des Sensors 2 die relativ kleinen Potentialschwankungen, die durch den elektrisch geladenen Faden während dessen Vorbeiflug am Fühler 5a auftreten. Eine abgeschirmte, elektrisch leitende Verbindung 5b führt das erfasste Signal einem Ladungsverstärker 10 zu. Die dem Ladungsverstärker 10 folgenden Elemente für die weitere Signalaufbereitung und Signalverarbeitung sind nicht dargestellt.

In Fig. 4 ist das Sensorelement 2 aus plattenförmigen Schichten aufgebaut. Eine mittlere, elektrisch leitende Schicht 5 ist zu beiden Seiten je mit einer isolierenden Schicht 6, 7 umgeben, auf welcher wiederum eine elektrisch leitende Schicht 3 oder 4 liegt. Die drei elektrisch leitenden Schichten 3, 4 und 5 sind dünne Metallschichten mit einer Dicke im Mikrometerbereich, wobei die äusseren Schichten 3 und 4 geerdet sind um die mit der Schicht 5 gebildeten elektrischen Leiter und Verbindungen vor äusseren elektrischen Feldern abzuschirmen. Die isolierenden Schichten 6 und 7 sind vorzugsweise wesentlich dicker als die elektrisch leitenden Schichten 3, 4 und 5, um eine steife mechanische Sandwichkonstruktion zu erhalten.

Das Sensorelement gemäss Fig. 3 ist derart ausgestaltet, dass es sich zwischen die Lamellen 23 des Webblattes 18 einfügen lässt und ohne weiteres Dicken von weniger als 1 mm aufweist. Die dem Schusskanal 19 des Profilblattes 18 zugewandte Kante des Sensorelementes 2 folgt den Konturen des Webblattes 18. Der schichtförmige Aufbau des Sensorelementes 2 ist z.B. mit einer mehrschichtigen Printplatine realisiert. Die mittlere elektrisch leitende Schicht 5 des Sensorelementes 2 besteht vorzugsweise aus einem Metall, wie z.B. Kupfer, und weist eine Dicke von wenigen Mikrometern auf. Aus dieser elektrisch leitenden Schicht 5 ist der eigentliche Fühler 5a, im vorliegenden Ausführungsbeispiel als offene Schlinge, ausgebildet, wobei der Fühler 5a den Schusskanal 19 innerhalb des Profilblattes 18 derart umfasst, dass der Querschnitt des Schusskanales 19 durch das Sensorelement 2 nicht vermindert wird. Der Fühler 5a kann natürlich durch unterschiedliche Formen ausgebildet sein, so z.B. nur durch die untere Hälfte der in Fig. 3 dargestellten Form.
Die mittlere Schicht 5 weist neben dem Fühler 5a eine leitende Verbindung 5b des Fühlers 5a zu einem Ladungsverstärker 10 auf. Aus der mittleren Schicht 5 können weitere leitende Verbindungen oder abschirmende, geerdete Schichten gebildet werden. Der Fühler 5a ist in Fig. 3 so angeordnet, dass die dem Schusskanal 19 zugewandte Seite gegen aussen elektrisch leitend ist. Der Fühler 5a kann auch derart in das Sensorelement 2 integriert werden, dass schusskanalseitig eine elektrisch isolierende Schicht zwischen dem Schusskanal 19 und dem Fühler 5a liegt. Der Fühler 5a wird dadurch unempfindlicher gegen Verschmutzung. Zur Sensitivitätserhöhung des Sensors kann auf der dem Schussfaden zugewendeten Kante ein elektrischer Leiter 24 aufgebracht sein, der nur mit dem Fühler 5a elektrisch leitend verbunden ist. Das Sensorelement 2 und der Träger 8 lassen sich zusammenhängend kombinieren, wenn beide Teile den gleichen plattenartigen schichtförmigen Aufbau aufweisen und somit ein einziges Teil bilden.

Fig. 5 zeigt eine Printplatine 13, aus deren mittleren leitenden Schicht 5 die Leiterbahnen 5c, die leitende Verbindung 5b sowie der Fühler 5a gebildet wurden. Die elektronischen Komponenten 9 sind vorzugsweise in SMD-Technik auf die Platine 13 aufgebracht, um Bohrungen für deren Anschlüsse zu vermeiden. Die integrierten Schaltkreise sind unmittelbar auf die isolierende Schicht 7 aufgebracht und mit den Leiterbahnen 5c verbunden. Für flache elektronische Komponenten, die innerhalb der Isolationsschicht 6 Platz finden, entsteht ein flacher Schussfadenwächter 1, dessen elektrische Komponenten 9, 10 durch die äusseren leitenden Schichten 3 und 4 elektrisch abgeschirmt sind. Die Leiterbahnen 5c könne auch aus einem Teilbereich der äussersten Schicht 4 des Trägers 8 gebildet werden. Die leitende Verbindung 5b wird zu den Leiterbahnen 5c durchkontaktiert. Die elektrische Abschirmung der elektronischen Komponenten 9, 10 ist durch zusätzliche elektrisch leitende Gehäusedeckel 11 und 12 erreicht. Eine elektrische Verbindung 16 führt das mindestens mit einem Ladungsverstärker 10 verstärkte Signal des Fühlers 5a nach aussen.

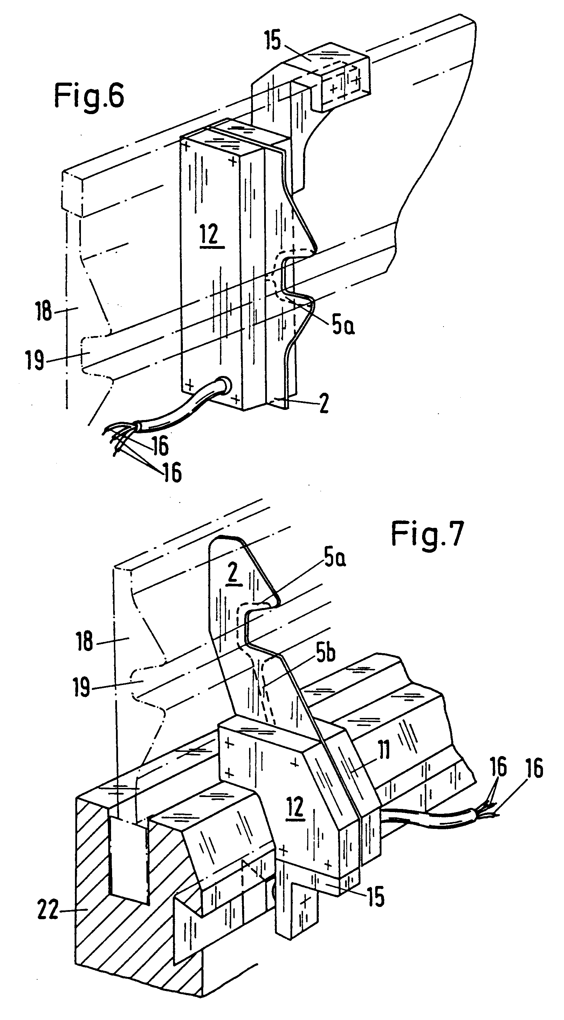

In Fig. 6 ist der Schussfadenwächter 1 ist mit Befestigungsmitteln 20 am Webblatt 18 oder an der Weblade 22 lösbar befestigt und durchdringt mit dem Sensor 5a die Lamellen. Er kann längs des Webblattes 18 variabel positioniert werden, wobei der Schussfadenwächter 1 sowie das Sensorelement 2 ausserhalb der Webbreite liegen und keine Kettfäden berühren.

Fig. 7 zeigt eine weitere Ausführungsform eines Schussfadenwächters 1 mit einer Befestigungsvorrichtung 15. Durch eine Verlängerung der leitenden Verbindung 5b zwischen Fühler 5a und dem Ladungsverstärker 10 ist die Form des Sensorelementes 2 derart ausgestaltet, dass das Sensorelement 2 schusskanalseitig zwischen die Lamellen 23 einfügbar ist und dass der Schussfadenwächter 1 schusskanalseitig an der Weblade 22, auch innerhalb der Webbreite, variabel positionierbar befestigt werden kann.

Fig. 8 zeigt eine weitere Ausführungsform eines Sensorelementes 2 mit einer Befestigungsvorrichtung 15, die sich auf der dem Schusskanal 19 abgewendeten Seite des Webblattes 18 an der Weblade 23, ebenfalls auch innerhalb der Webbreite, variabel positionierbar befestigen lässt.

Die Befestigungsvorrichtung 15 weist die zusätzliche Eigenschaft auf, dass Vorrichtungen vorhanden sind, die es erlauben, den Fühler 5a im Schusskanal 19 des Webblattes 18 derart zu positionieren, dass das Sensorelement 2 den Schusskanal 19 mindestens teilweise anfasst, ohne in den Schusskanal 19 hineinzuragen. Der für Luftwebmaschinen beschriebene Schussfadenwächter 1 eignet sich auch für andere Webmaschinentypen, bei denen der Faden eine elektrostatische Ladung aufweist. So ist dieser Schussfadenwächter z.B. auch für Projektilwebmaschinen geeignet.

## Patentansprüche

1. Schussfadenwächter für Webmaschinen, dessen Sensorelement (2) berührungslos auf elektrische Ladung eines Schussfadens anspricht bzw. empfindlich ist, dadurch gekennzeichnet, dass das Sensorelement (2) aus plattenartigen Schichten aufgebaut ist und mindestens drei leitende, durch Isolationsmaterial (6, 7) getrennte, Schichten (3, 4, 5) ausweist, wobei die äusseren Schichten (3, 4) geerdet sind und die mittlere Schicht (5) zusammen mit weiteren Elementen einen Fühler (5a) zur Detektion der elektrischen Ladung des Schussfadens bildet.

2. Schussfadenwächter nach Anspruch 1, dadurch gekennzeichnet, dass die Schichten (3, 4, 5, 6, 7) des Sensorelementes (2) vorzugsweise rechtwinklig zur Eintragsrichtung des Schussfadens angeordnet sind und dass die dem Schussfaden zugewendete Kante der Schichten (3, 4, 5, 6, 7) die Konturen des Schusskanals eines Webblattes (18) aufweist.

3. Schussfadenwächter nach einem der Ansprüche 1 oder 2 dadurch gekennzeichnet, dass der mit der mittleren, leitenden Schicht (5) des Sensorelementes (2) gebildete Fühler (5a) die Form einer offenen Schlinge aufweist.

4. Schussfadenwächter nach einem der Ansprüche 1 bis 3 dadurch gekennzeichnet, dass der Fühler (5a) derart im Sensorelement (2) integriert ist, dass an der dem Schussfaden zugewendeten Kante eine elektrisch isolierende Schicht zwischen dem Schusskanal (19) und dem Fühler (5a) liegt.

5. Schussfadenwächter nach einem der Ansprüche 1 bis 3 dadurch gekennzeichnet, dass der Fühler (5a) an der dem Schussfaden zugewendeten Kante elektrisch freigelegt ist.

6. Schussfadenwächter nach einem der Ansprüche 1 bis 3, dadurch gekennzeichnet, dass auf der dem Schussfaden zugewendeten Kante ein elektrischer Leiter (24) aufgebracht ist, der nur mit dem Fühler (5a) elektrisch leitend verbunden ist.

7. Schussfadenwächter nach einem der Ansprüche 1 bis 6 dadurch gekennzeichnet, dass das Sensorelement (2) aus einer mehrschichtigen Printplatine, insbesondere in Multilayertechnik, gebildet ist.

8. Schussfadenwächter nach einem der Ansprüche 1 bis 7 dadurch gekennzeichnet, dass der Fühler (5a) mit einem Ladungsverstärker (10) verbunden ist, wobei die elektrische Verbindung (5b) zum Ladungsverstärker (10) durch die leitenden, äusseren Schichten (3,4) des Sensorelementes (2) elektrisch abgeschirmt ist.

9. Schussfadenwächter nach einem der Ansprüche 1 bis 8 dadurch gekennzeichnet, dass ein Träger (8) den gleichen schichtförmigen Aufbau wie das Sensorelement (2) aufweist und zusammen mit dem Sensor eine Einheit bildet, in der die mittlere, leitende Schicht (5) des Sensorelementes (2) aus einer Printplatine (13) mit Fühler (5a), leitender Verbindung (5b) und Leiterbahnen (5c) zum Aufbringen eines Ladungsverstärkers (10) und/oder weiteren elektronischen Komponenten (9) besteht.

10. Schussfadenwächter nach einem der Ansprüche 1 bis 9 dadurch gekennzeichnet, dass die elektronischen Komponenten (9), insbesondere der Ladungsverstärker (10), direkt auf die Printplatine (13) aufgebracht und innerhalb der elektrisch isolierenden Schicht (6) oder (7) angeordnet sind, so dass die leitenden Schichten (3) und (4) ausser schusskanalseitig den gesamten Schussfadenwächter (1) elektrisch abschirmen.

11. Schussfadenwächter nach einem der Ansprüche 1 bis 8 dadurch gekennzeichnet, dass ein Träger (8) den gleichen schichtförmigen Aufbau wie das Sensorelement (2) aufweist und zusammen mit dem Sensor eine Einheit bildet, und dass ein mit einem Gehäusedeckel (12) gegen aussen elektrisch abgeschirmter Teilbereich der äusseren Schicht (4) des Trägers (8) Leiterbahnen (5c) zum Aufbringen eines Ladungsverstärkers (10) und/oder weiteren elektronischen Komponenten (9) bildet, wobei die leitende Verbindung (5b) zu den Leiterbahnen (5c) durchkontaktiert ist.

12. Webmaschine mit einem Schussfadenwächter nach einem der Ansprüche 1 bis 11.

13. Webmaschine nach Anspruch 12 dadurch gekennzeichnet, dass der Schussfadenwächter (1) mindestens teilweise zwischen Lamellen (23) des Webblattes (18) einfügbar ist.

14. Webmaschine nach einem der Ansprüche 12 oder 13 dadurch gekennzeichnet, dass der Schussfadenwächter (1) mit einer Befestigungsvorrichtung (15) am Webblatt (18) oder an der Weblade (22) befestigt ist.

15. Webmaschine nach einem der Ansprüche 12 bis 14 dadurch gekennzeichnet, dass der Schussfadenwächter (1) längs des Webblattes (18) variabel positionierbar ist und dass das Sensorelement ausserhalb der Webbreite liegt.

## Claims

1. A weft detector for looms, in which the sensor element (2) reacts or is sensitive without contact to electrical charge of a weft thread,
**characterised in that** the sensor element (2) is constructed of plate-type layers and comprises at least three conductive layers (3, 4, 5) separated by insulation material (6, 7), with the outer layers (3, 4) being earthed and the middle layer (5) together with other elements forming a feeler (5a) for detecting the electrical charge of the weft thread.

2. A weft detector according to Claim 1,
**characterised in that** the layers (3, 4, 5, 6, 7) of the sensor element (2) are preferably disposed at right angles to the direction of insertion of the weft thread,
**and in that** the edge of the layers (3, 4, 5, 6, 7) close to the weft thread has the contours of the weft channel of a reed (18).

3. A weft detector according to one of Claims 1 or 2,
**characterised in that** the feeler (5a) formed with the middle conductive layer (5) of the sensor element (2) has the form of an open loop.

4. A weft detector according to one of Claims 1 to 3,
**characterised in that** the feeler (5a) is integrated in the sensor element (2) in such a way that on the edge close to the weft thread an electrically insulating layer lies between the weft channel (19) and the feeler (5a).

5. A weft detector according to one of Claims 1 to 3,
**characterised in that** the feeler (5a) is electrically uncovered on the edge close to the weft thread.

6. A weft detector according to one of Claims 1 to 3,
**characterised in that** an electrical conductor (24), which is only connected in an electrically conductive way to the feeler (5a), is mounted on the edge close to the weft thread.

7. A weft detector according to one of Claims 1 to 6,
**characterised in that** the sensor element (2) is formed from a multi-layer printed circuit board, in particular in multi-layer technique.

8. A weft detector according to one of Claims 1 to 7,
**characterised in that** the feeler (5a) is connected to a charge amplifier (10), with the electrical connection (5b) to the charge amplifier (10) being electrically screened by the conductive outer layers (3, 4) of the sensor element (2).

9. A weft detector according to one of Claims 1 to 8,
**characterised in that** a support (8) comprises the same laminated construction as the sensor element (2) and together with the sensor forms a unit, in which the middle conductive layer (5) of the sensor element (2) consists of a printed circuit board (13) having feeler (5a), conductive connection (5b) and strip conductors (5c) to supply a charge amplifier (10) and/or further electronic components (9).

10. A weft detector according to one of Claims 1 to 9,
**characterised in that** the electronic components (9), especially the charge amplifier (10), are directly mounted on the printed circuit board (13) and are disposed inside the electrically insulating layer (6) or (7), so that the conductive layers (3) and (4) electrically screen the entire weft detector (1) except on the side of the weft channel.

11. A weft detector according to one of Claims 1 to 8,
**characterised in that** a support (8) has the same laminated construction as the sensor element (2) and together with the sensor forms a unit,
**and in that** a sub-region of the outer layer (4) of the support (8) electrically screened with respect to the outside with a housing lid (12) forms strip conductors (5c) for supplying a charge amplifier (10) and/or further electronic components (9), with the conductive connection (5b) being through-hole plated to the strip conductors (5c).

12. A loom having a weft detector as specified in one of Claims 1 to 11.

13. A loom according to Claim 12,
**characterised in that** the weft detector (1) can be inserted at least partially between drop wires (23) of the reed (18).

14. A loom according to one of Claims 12 or 13,
**characterised in that** the weft detector (1) is attached with an attachment device (15) to the reed (18) or to the loom sley (22).

15. A loom according to one of Claims 12 to 14,
**characterised in that** the weft detector (1) can be positioned variably along the reed (18),
**and in that** the sensor element lies outside the loom width.

## Revendications

1. Détecteur de fil de trame pour métiers à tisser, dont l'élément de détection (2) réagit ou est sensible sans contact à la charge électrique d'un fil de trame, caractérisé en ce que l'élément de détection (2) est constitué de couches en forme de plaque et présente au moins trois couches (3, 4, 5) conductrices séparées par un matériau d'isolation (6, 7), tandis que les couches extérieures (3, 4) sont mises à la terre et que la couche médiane (5) forme avec d'autres éléments une sonde (5a) de détection de la charge électrique du fil de trame.

2. Détecteur de fil de trame selon la revendication 1, caractérisé en ce que les couches (3, 4, 5, 6, 7) de l'élément de détection (2) sont disposées de préférence à angle droit par rapport à la direction d'insertion de la duite, et en ce que le bord des couches (3, 4, 5, 6, 7) tourné vers le fil de trame présente les contours du canal de duite d'un peigne (18).

3. Détecteur de fil de trame selon l'une des revendications 1 ou 2, caractérisé en ce que la sonde (5a) formée par la couche médiane (5), conductrice, de l'élément de détection (2), présente la forme d'une boucle ouverte.

4. Détecteur de fil de trame selon l'une des revendications 1 à 3, caractérisé en ce que la sonde (5a) est intégrée dans l'élément de détection (2) de telle sorte qu'une couche électriquement isolante soit située entre le canal de duite (19) et la sonde (5a), sur le bord tourné vers le fil de trame.

5. Détecteur de fil de trame selon l'une des revendications 1 à 3, caractérisé en ce que la sonde (5a) est électriquement libre sur le bord tourné vers le fil de trame.

6. Détecteur de fil de trame selon l'une des revendications 1 à 3, caractérisé en ce qu'un conducteur électrique (24) qui n'est relié électriquement qu'avec la sonde (5a) est installé sur le bord tourné vers le fil de trame.

7. Détecteur de fil de trame selon l'une des revendications 1 à 6, caractérisé en ce que l'élément de détection (2) est formé de plusieurs plaques imprimées en plusieurs couches, en particulier en technologie multicouches.

8. Détecteur de fil de trame selon l'une des revendications 1 à 7, caractérisé en ce que la sonde (5a) est reliée à un amplificateur de charge (10), tandis que la liaison électrique (5b) avec l'amplificateur de charge (10) est blindée électriquement par les couches extérieures conductrices (3, 4) de l'élément de détection (2).

9. Détecteur de fil de trame selon l'une des revendications 1 à 8, caractérisé en ce qu'un support présente la même structure en forme de couches que l'élément de détection (2) et forme avec le détecteur un module dans lequel la couche médiane conductrice (5) de l'élément de détection (2) est constituée d'une plaque imprimée (13) comportant une sonde (5a), une liaison conductrice (5b) et des parcours conducteurs (5c) pour l'installation d'un amplificateur de charge (10) et/ou d'autres composants électroniques (9).

10. Détecteur de fil de trame selon l'une des revendications 1 à 9, caractérisé en ce que les composants électroniques (9), en particulier l'amplificateur de charge (10), sont installés directement sur la plaque imprimée (13) et sont disposés à l'intérieur de la couche électriquement isolante (6) ou (7), de sorte que les couches conductrices (3) et (4) protègent électriquement l'ensemble du détecteur de fil de trame en dehors du côté tourné vers le canal de duite.

11. Détecteur de fil de trame selon l'une des revendications 1 à 8, caractérisé en ce qu'un support (8) présente la même structure en couches que l'élément de détection (2) et forme avec le détecteur un module, et en ce qu'une partie de la couche extérieure (4) du support (8), blindée électriquement vis-à-vis de l'extérieur par un couvercle de boîtier (12), forme des parcours conducteurs (5c) pour l'installation d'un amplificateur de charge (10) et/ou d'autres composants électroniques (9), tandis que la liaison conductrice (5b) est mise en contact avec les parcours conducteurs (5c).

12. Métier à tisser comportant un détecteur de fil de trame selon l'une des revendications 1 à 11.

13. Métier à tisser selon la revendication 12, caractérisé en ce que le détecteur de fil de trame (1) peut être inséré au moins partiellement entre des lamelles (23) du peigne (18).

14. Métier à tisser selon l'une des revendications 12 ou 13, caractérisé en ce que le détecteur de fil de trame (1) peut être fixé par un dispositif de fixation (15) sur le peigne (18) ou sur le battant de tissage (22).

15. Métier à tisser selon l'une des revendications 12 à 14, caractérisé en ce que le détecteur de fil de trame (1) peut être placé en différentes positions le long du peigne (18), et en ce que l'élément de détection est situé à l'extérieur de la largeur de tissage.
